# EUROPÄISCHE PATENTSCHRIFT

(11) **EP 1 102 579 B1**
(45) Veröffentlichungstag und Bekanntmachung des Hinweises auf die Patenterteilung: **19.03.2003**
(21) Anmeldenummer: 99932599.6
(22) Anmeldetag: 02.08.1999
(51) Int. Cl.: A61K 9/12, A61K 47/22

(54) **MEDIZINISCHE AEROSOLFORMULIERUNGEN**
MEDICINAL AEROSOL FORMULATIONS
FORMULATIONS D'AEROSOL A USAGE MEDICAL

(30) Priorität: 04.08.1998 CH 163398
(43) Veröffentlichungstag der Anmeldung: 30.05.2001
(73) Patentinhaber: JAGO RESEARCH AG, CH-4132 Muttenz (CH)
(72) Erfinder: KELLER, Manfred, D-79189 Bad Krozingen (DE); HERZOG, Kurt, CH-4055 Basel (CH); MÜLLER-WALZ, Rudi, D-79650 Schopfheim (DE); KRAUS, Holger, CH-4462 Rickenbach (CH)
(74) Vertreter: Zimmermann, Hans, Dr.
(86) Internationale Anmeldenummer: CH9900360
(87) Internationale Veröffentlichungsnummer: WO00007567

(56) Entgegenhaltungen:
- WO-A-92/00061
- DE-A- 4 003 272
- FR-A- 2 339 604

## Beschreibung

Die vorliegende Erfindung betrifft medizinische Suspensionsaerosolformulierungen sowie eine neue Verwendung von Cromoglicinsäure- und Nedocromil-Salzen.

Zur Herstellung medizinischer Dosieraerosole eignen sich in der Regel nur Treibgase, die sich bei Raumtemperatur verflüssigen lassen und die bei sukzessivem Absprühen des Inhalts zu keiner oder nur allenfalls zu einer geringfügigen Abnahme des Binnendrucks im Behältnis führen. In der Vergangenheit wurden üblicherweise Fluorchlorkohlenwasserstoffe (FCKWs), wie z.B Trichlortrifluormethan (F11), Dichlordifluormethan (F12) und 1,2-Dichlor-1,1,2,2-tetrafluorethan (F114), und gelegentlich auch kurzkettige Alkane, wie z.B. Propan, Butan und Isobutan, verwendet.

Aufgrund der Ozonproblematik, hervorgerufen durch die Abspaltung von radikalischen Chloratomen aus den FCKWs, haben sich im Montrealer Abkommen viele Staaten darauf verständigt, die FCKWs als Treibmittel zukünftig nicht mehr zu verwenden. Gase, wie Kohlendioxid, Stickstoff und dergleichen, lassen sich zwar unter Druck verflüssigen, sind aber als Treibmittel für Dosieraerosole nicht brauchbar, weil der Binnendruck im Behältnis mit zunehmender Entleerung sehr stark abnimmt. Hingegen haben sich fluorierte Alkane, insbesondere Hydrofluoralkane (im Rahmen der vorliegenden Erfindung auch als "HFA" bezeichnet) wie 1,1,1,2-Tetrafluorethan (HFA 134a) und 1,1,1,2,3,3,3-Heptafluorpropan (HFA 227), als geeignete FCKW-Ersatzstoffe für den medizinischen Bereich erwiesen, da sie inert sind und eine sehr geringe Toxizität aufweisen. Aufgrund ihrer physikalischen Eigenschaften, wie Druck, Dichte etc. sind sie besonders geeignet, um FCKWs wie F11, F12 und F114 als Treibmittel in Dosieraerosolen zu ersetzen.

Es hat sich jedoch gezeigt, dass die üblichen, in FCKW-haltigen Dosieraerosolen verwendeten Hilfsstoffe, wie Lecithin, Sorbitantrioleat und Ölsäure, in Hydrofluoralkanen, wie HFA 134 und HFA 227, nur unzureichend löslich sind. Es wurde daher vorgeschlagen, entweder in an sich bekannter Weise die Löslichkeit durch Verwendung eines Cosolvens wie Ethanol zu verbessern oder die Wirkstoffteilchen mit dem oberflächenaktiven Mittel zu überziehen oder - statt dessen - auf ein oberflächenaktives Mittel zu verzichten oder spezielle, treibgaslösliche oberflächaktive Mittel zu verwenden. Solche Vorschläge finden sich beispielsweise in US-A-2 868 691, US-A-3 014 844, DE-A-2 736 500, EP-A-0 372 777, WO-A-91/11495, EP-A-0 504 112, EP-B-0 550 031, WO-A-91/04011, EP-A-0 504 112 und WO-A-92/00 061.

Es ist allgemein bekannt, dass im Falle von Suspensionsformulierungen nur Wirkstoffteilchen, die kleiner als 6 µm sind, lungengängig sind. Zur gewünschten Deposition der Wirkstoffe in der Lunge, müssen diese deshalb vor der Verarbeitung mittels spezieller Verfahren, wie z.B. Stift-, Kugel- oder Luftstrahlmühlen zerkleinert bzw. mikronisiert werden. Ein Mahlprozess führt aber in der Regel zu einer Oberflächenvergrösserung, die mit einer Erhöhung der elektrostatischen Ladung des mikronisierten Wirkstoffes einhergeht, wodurch dann meistens das Fliessverhalten und die Wirkstoff-Dispergierung verschlechtert wird. Als Folge der Grenz- und Ladungsaktivitäten kommt es häufig zu einer Agglomeration von Wirkstoffpartikeln oder auch zur Adsorption von Wirkstoff an Grenzflächen, die z.B. in der Anlagerung an Geräte oder Behälteroberflächen augenfällig wird.

In Aerosolzubereitungen, bei denen der Wirkstoff in verflüssigtem Treibgas suspendiert vorliegt, kann es zu einer Deposition bzw. Ringbildung im Behälter an der Stelle kommen, wo die Flüssigphase in die Gasphase übergeht. Ohne Benetzung der mikronisierten Wirkstoffpartikel oder Abführen von Ladungen sowie Modifikation ihrer Oberflächeneigenschaften lassen sich Suspensionen nur unzureichend stabilisieren bzw. in dispergiertem Zustand halten. Die mangelhafte Benetzung bzw. Dispergierung der Wirkstoffpartikel hat auch zur Folge, dass diese in vielen Fällen eine hohe Adsorptionstendenz aufweisen und an Oberflächen, wie z.B. der Behälterinnenwand oder dem Ventil kleben, was dann zu einer Unterdosierung sowie einer schlechten Dosiergenauigkeit von Sprühstoss zu Sprühstoss führt. Bei Suspensionen ist es deshalb in der Regel erforderlich, einen oberflächenaktiven Hilfsstoff oder ein Gleitmittel zuzusetzen, um die Adsorption an Grenzflächen zu erniedrigen und eine akzeptable Dosiergenauigkeit zu erreichen. Besonders problematisch ist eine im Laufe der Lagerung eintretende Veränderung bzw. Erniedrigung des Anteils der inhalierbaren, lungengängigen Teilchen, der sogenannten Fine Particle Dose (FPD), was zu einer Abnahme der Wirksamkeit der Aerosolformulierung führt.

Zur Überwindung dieser Probleme werden in der Regel zugelassene oberflächenaktive Substanzen zugesetzt, wie sie bereits früher bei den FCKW-haltigen Formulierungen Anwendung fanden. Alternativ dazu kann in gewissen Fällen eine Modifikation der Oberflächeneigenschaften durch verschiedene Massnahmen (z.B. Coating) helfen, diese unerwünschten Effekte zu minimieren. Weil sich aber oberflächenaktive Mittel wie Ölsäure, Sorbitantrioleat und Lecithin nur unzureichend in Hydrofluoralkanen wie HFA 134a und/oder HFA 227 lösen, wird bzw. muss ein polares Cosolvens wie z.B. Ethanol zugesetzt werden, damit man die pharmazeutisch-technologischen Probleme besser beherrschen kann.

Wird allerdings Ethanol in höherer Konzentration zugesetzt, erniedrigt sich die Dichte der Treibgasmischung, was vor allem bei Suspensionen zu einer unerwünschten Entmischung führen kann. Man kann zudem unerwünschterweise einen "nassen Spray" erhalten, weil das Treibgas viel schneller verdampft als Ethanol. Daneben kann es aber durch die Erhöhung der Löslichkeit während der Lagerung auch zu Anlösungseffekten kommen, was dann zu einem Kristallwachstum und wiederum zu einer Erniedrigung der Menge an inhalierbaren, lungengängigen Teilchen, der sogenannten Fine Particle Dose (FPD), führt.

Nachteilig ist des weiteren, dass bei Ethanolkonzentrationen von z.B. 10% oder mehr, der Anteil inhalierbarer Partikel (< 6 µm) abnimmt, weil der Spray aufgrund der andersgearteten Verdampfungseigenschaften von Ethanol zum Treibgas, Teilchen mit grösserem aerodynamischem Durchmesser ergeben kann. Als Folge davon kommt es zu einer Erniedrigung der für die Wirksamkeit entscheidenden Fine Particle Dose (FPD). Dies mag erklären, weshalb die meisten handelsüblichen Dosieraerosole als Suspensionen formuliert wurden.

Bei ethanolhaltigen. Aerosolen kann es zudem auch im Falle von Suspensionsformulierungen gelegentlich zu Problemen der Wirkstoffstabilität kommen. Ferner kann die Wirkstoffstabilität, die Wirkstoff-Dispergierung und die Fine Particle Dose auch. durch Feuchtigkeit beeinträchtigt werden.

Zur Messung der aerodynamischen Partikelgrössenverteilung bzw. der FPD oder Fine Particle Fraction (FPF) eignen sich Impaktoren, wie z.B. der 5-Stufen Multistage Liquid Impinger (MSLI) oder 8-Stufen Andersen Kaskaden Impaktor (ACI), die in Chapter <601> der United States Pharmacopoeia (USP) oder in der Inhalanda Monographie der Europäischen Pharmacopoe (Ph. Eur.) beschrieben sind. Anhand der aerodynamischen Partikelverteilung kann man mittels eines sogenannten "Log-probability plots" (logarithmische Darstellung der Wahrscheinlichkeitsverteilung) den mittleren aerodynamischen Teilchendurchmesser (Median Mass Aerodynamic Diameter MMAD) von Aerosol-Zubereitungen berechnen. Mit diesen Informationen zur Partikelverteilung erhält man Informationen, ob der Wirkstoff eher im oberen oder unteren Lungenbereich deponiert wird.

Wie sich aus dem Vorangehenden ergibt, stellt die Einhaltung einer hohen Dosiergenauigkeit, d.h. die gleichbleibende Wirkstofffreigabe von Sprühstoss zu Sprühstoss, ein grundsätzliches Problem der Suspensions-Dosieraerosole dar, das durch den Ersatz der FCKWs noch zusätzlich erschwert wird. Die Dosiergenauigkeit hängt neben dem Ventil und Actuator im wesentlichen von den Suspensionseigenschaften ab, d.h. davon, wie gut und homogen der Wirkstoff im Treibmittel dispergiert ist und wie lange die Suspension ohne Änderung ihrer physikalischen Eigenschaften in diesem labilen Gleichgewichtszustand verbleibt. Als besonders schwierig erweist sich die Einhaltung einer hohen Dosiergenauigkeit im Falle potenter, niedrig dosierter Wirkstoffe. Beispielsweise benötigt man für den langwirkenden Beta-agonisten Formoterol-fumarat, der bereits in sehr geringen Dosen (6 µg/Hub) therapeutisch wirksam ist, eine Formulierung, die eine hinreichend stabile Suspension ergibt, die nicht an Grenzflächen klebt und sich im Laufe der Lagerung bei unterschiedlichen Temperatur- und Feuchtebedingungen nicht verändert. Ein Überblick der am Markt befindlichen Produkte zeigt, dass es bis heute kein Dosieraerosol gibt, das Wirkstoffe in Mengen von weniger als 10 µg pro Hub (d.h. pro Strühstoss) mit einer Streuung besser als ± 25% dosieren kann.

DE-A-4 003 272 offenbart Aerosol formulierungen mit nicht Spezifizierter Menge an Chromglicinsäure.

Der Erfindung liegt daher die Aufgabe zugrunde, die erwähnten Probleme von Suspensions-Dosieraerosolen möglichst weitgehend zu vermeiden und insbesondere eine medizinische Suspensionsaerosolformulierung bereitzustellen, die verbesserte Suspensions- und Haltbarkeitseigenschaften aufweist, den nachteiligen Effekt von Wasser auf die Stabilität und Dispergierung von Wirkstoffen weitestgehend unterbindet und eine hohe Dosiergenauigkeit - auch bei niedrig dosierten Wirkstoffen - gestattet.

Die Aufgabe wird erfindungsgemäss gelöst durch eine medizinische Aersolformulierung, umfassend ein festes, pharmazeutisch annehmbares Salz von Cromoglicinsäure und/oder Nedocromil in therapeutisch und prophylaktisch unwirksamer Menge, eine wirksame Menge eines davon verschiedenen, fein verteilten pharmazeutischen Wirkstoffes mit einem mittleren Teilchendurchmesser von weniger als 6 µm und ein nicht toxisches flüssiges Treibmittel, in dem der Wirkstoff in suspendierter Form vorliegt.

Es wurde nämlich überraschenderweise gefunden, dass die Zugabe von Cromoglicinsäure- und/oder Nedocromilsalzen in subtherapeutischen Konzentrationen von beispielsweise 5 - 100 µg/Hub sehr hilfreich ist, um pharmazeutische Wirkstoffe in Hydrofluoralkanen und anderen Treibmitteln zu suspendieren. Diese Substanzen, sogenannte Mastzellinhibitoren, werden bislang in Form ihrer Natriumsalze in topischen Präparaten (Augentropfen, Nasensprays) als auch in Inhalationsprodukten als Antiallergika eingesetzt. Beispielsweise offenbart FR-A-2 339 604 Dinatriumcromoglykat mit einem Wassergehalt von weniger als 5 Gew.-% sowie Aerosolformulierungen mit einem Wassergehalt von weniger als 1 Gew.-%, die 1-20 Gew.-% an fein verteiltem Dinatriumcromoglykat und als Treibmittel vorzugsweise FCKWs wie F11, F12 und F114 enthalten. In WO-A-91/11495 sind Treibgasmischungen, die ein teilfluoriertes niederes Alkan wie HFA 227, HFA 125, HFA 134a oder HFA 152a enthalten, sowie Pulveraerosole auf der Basis solcher Treibgasmischungen beschrieben, wobei als Beispiele geeigneter Arneistoffe u.a. Antiallergika wie Dinatriumcromoglykat und Nedocromil und ferner auch Wirkstoffkombinationen von Dinatriumcromoglykat mit Betamimetika bzw. PAF-Antagonisten genannt werden. Aus WO-A-92/00061 sind Aerosolformulierungen bekannt, umfassend ein Fluorkohlenwasserstoff-Treibmittel, ein polyethoxyliertes oberflächenaktives Mittel und ein Medikament, wobei als Medikament vorzugsweise das Salz einer Dicarbonsäure, beispielsweise ein Salz von Nedocromil oder Cromoglicinsäure, verwendet werden kann. In den vorbekannten Formulierungen werden die Cromoglicinsäure- oder Nedocromilsalze aber jeweils in inhalierbarer Form und in therapeutisch und/oder prophylaktisch wirksamer Konzentration eingesetzt.

In kommerziellen Anwendungen wird Dinatriumcromoglykat in Dosieraerosolen in Konzentrationen von 1 mg und 5 mg/Hub eingesetzt und in Inhalationslösungen und -pulvern in Konzentrationen von 20 mg pro Applikation. Solche Produkte sind unter dem Handelsnamen Intal® (Fisons) erhältlich und werden auch als Generika von verschiedenen Firmen angeboten. Nedocromil-Natrium wird als Tilade® Dosieraerosol (Fisons) in einer Konzentration von 2 mg/Hub therapeutisch eingesetzt. Daneben gibt es auch Kombinationsprodukte aus dem Antiallergikum Dinatriumcromoglykat und einem Betaagonisten, die pro Sprühstoss 1 mg Dinatriumcromoglykat und z.B. 0,5 mg Reproterol-Hydrochlorid (Aarane® von Fisons, Allergospasmin® von Asta Medica) oder 0,05 mg Fenoterol-Hydrobromid (Ditec® von Thomae) enthalten.

Überraschenderweise wurde ferner festgestellt, dass anstelle bekannter inhalationszugelassener Trägerstoffe, wie z.B. Lactose oder Glucose, die hochdosierten Wirkstoffe Cromoglicinsäure und Nedocromil als Trägerstoffe verwendet werden können, wenn diese in Form ihrer Salze in subtherapeutischen Dosen eingesetzt werden. Pharmazeutisch annehmbare Salze beider Substanzen eignen sich dazu, andere Wirkstoffe zu schützen und Dosieraerosole mit vorteilhaften Produkteigenschaften zu erhalten. Wird ein pharmazeutischer Wirkstoff wie z.B. Formoterol-fumarat mit einem pharmazeutisch annehmbaren Salz von Cromoglicinsäure und/oder Nedocromil als Träger gemischt, erhält man ein Pulvergemisch, das sich in den üblichen Treibmitteln meist auch ohne ein oberflächenaktives Mittel gut suspendieren lässt. Die erfindungsgemässen anionischen Trägermaterialien, die z.B. in Form ihrer Natriumsalze vorliegen können, bilden aufgrund ihrer chemischen Ladungen mit Wirkstoffen, wie z.B. Formoterol-fumarat, Levalbuterolsulfat und dergleichen Assoziate, die auch in sehr niedrigen Dosierungen genau dosiert werden können.

Überraschenderweise wurde des weiteren gefunden, dass Salze von Cromoglicinsäure und Nedocromil, wie Dinatriumcromoglykat und Nedocromil-Natrium, die chemische bzw. physikalische Stabilität von feuchtigkeitsempfindlichen Wirkstoffen, wie z.B. Levalbuterol-sulfat, Formoterol-fumarat und dergleichen, verbessern, was möglicherweise auf ihre Hygroskopizität zurückzuführen sein könnte, indem sie gewissermassen den negativen Einfluss des Wassers auf feuchtigkeitsempfindliche Wirkstoffen fernhalten.

Die Adhäsionstendenz von elektrostatisch aufgeladenen Wirkstoffen wie z.B. mikronisierten Corticosteroiden wird zudem durch Zumischung von Salzen von Cromoglicinsäure und/oder Nedocromil, wie z.B. Dinatriumcromoglykat und/oder Nedocromil-Natrium, reduziert und ihre Dispergierungseigenschaften werden verbessert.

Die erfindungsgemässe Verwendung von pharmazeutisch annehmbaren Salzen von Cromoglicinsäure und/oder Nedocromil als Trägermaterialien in therapeutisch und prophylaktisch unwirksamer Menge gestattet daher die Herstellung verbesserter Suspensionsaerosolformulierungen, wobei auf Hilfsstoffe wie Ölsäure, Sorbitantrioleat, Lecithin, Lactose und Glucose, die sich in Hydrofluoralkanen wie HFA 134a und HFA 227 nur unzureichend oder gar nicht lösen, gewünschtenfalls ganz oder weitgehend verzichtet werden kann. Ein weiterer Vorteil der erfindungsgemäss verwendeten Trägermaterialien besteht darin, dass die Natriumsalze bereits als Wirkstoffe in höherer Dosierung zugelassen sind und sich damit aufwendige Sicherheitsprüfungen zum Nachweis der ihrer Unbedenklichkeit erübrigen.

Die erfindungsgemässe Aerosolformulierung eignet sich grundsätzlich für beliebige als Supensionsaerosole applizierbare pharmazeutische Wirkstoffe in jeweils therapeutisch oder prophylaktisch wirksamer Menge. Beispiele bevorzugter Wirkstoffe sind Betamimetika, Anticholinergika, Antiallergika und entzündungshemmende Wirkstoffe (z.B. Corticosteroide, Leukotrienantagonisten, Cytokininhibitoren, Kaliumkanalöffner etc.). Besonders bevorzugt sind Formulierungen, die als pharmazeutischen Wirkstoff Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid oder ein pharmazeutisch annehmbares Salz oder Derivat dieser Wirkstoffe, wie z.B. Formoterol-fumarat, Formoterol-tartrat, Salmeterol-xinafoat, Fenoterol-Hydrobromid, Clenbuterol-Hydrochlorid, Ipratropiumbromid, Oxitropiumbromid, Glycopyrroniumbromid, Tiotropiumbromid, Mometason-furoat, Fluticason-dipropionat, Beclomethason-dipropionat oder Flunisolidacetat, enthalten, wobei optische aktive Wirkstoffe in Form ihres wirksamen Isomers oder als Isomerengemisch (z.B. Racemat) verwendet werden können. Gewünschtenfalls können die erfindungsgemässen Aerosolformulierungen auch zwei oder mehrere pharmazeutisch Wirkstoffe enthalten, wobei Kombinationen von Fluticason, Ipratropium, Oxitropium, Glycopyrronium, Tiotropium, Budesonid, Mometason, Ciclesonid, Rofleponid oder einem pharmazeutisch annehmbaren Salz oder Derivat davon mit Levalbuterol, Formoterol und/oder Salmeterol oder einem pharmazeutisch annehmbaren Derivat davon bevorzugt sind. Gewünschtenfalls können die erfindungsgemässen Aerosolformulierungen auch gelöste Wirkstoffe enthalten; erfindungswesentlich ist lediglich, dass mindestens ein pharmazeutischer Wirkstoff in supendierter Form vorliegt. Wie sich aus den nachfolgenden Erläuterungen ergibt, werden jedoch pharmazeutisch annehmbare Cromoglicinsäure- und Nedocromilsalze erfindungsgemäss nur als Träger verwendet, d.h. der "pharmazeutische Wirkstoff" ist im Rahmen der vorliegenden Erfindung kein pharmazeutisch annehmbares Salz von Cromoglicinsäure oder Nedocromil.

Die erfindungsgemässe Aerosolformulierung ist besonders vorteilhaft für die Verabreichung niedrig dosierter Wirkstoffe. Die Erfindung betrifft daher insbesondere auch Aerosolformulierungen von Wirkstoffen, die in einer wirksamen Dosis von etwa 0,1 bis 100 µg pro Sprühstoss verabreicht werden können, wobei solche mit einer Dosis von höchstens etwa 50 µg bevorzugt und solche mit einer Dosis von etwa 0,1 bis 20 µg besonders bevorzugt sind. Die Hubmassen handelsüblicher MDIs (Metered Dose Inhalers) liegen meist im Bereich von etwa 30 bis 130 mg (mit Ventilen entsprechend etwa 25 bis 100 µl) und typischerweise bei etwa 70 mg pro Sprühstoss. Dementsprechend enthalten die bevorzugten Aerosolformulierungen niedrig dosierter Wirkstoffe in der Regel etwa 0,0001 bis 0,2 Gew.-%, insbesondere höchstens etwa 0,1 Gew.-% und besonders bevorzugt etwa 0,0001 bis 0,04 Gew.-% an suspendiertem Wirkstoff.

Der zu suspendierende Wirkstoff bzw. die zu suspendierenden Wirkstoffe können in an sich bekannter Weise, z.B. mittels Stift-, Kugel- oder Luftstrahlmühlen, mikronisiert oder durch kontrollierte Mikrokristallisation oder Fällung erhalten und im Treibmittel suspendiert werden. Zweckmässigerweise soll der mittlere Teilchendurchmesser der Wirkstoffteilchen weniger als 6 µm und vorzugsweise mindestens etwa 1 µm betragen, wobei der "mittlere Teilchendurchmesser" im Rahmen der vorliegenden Erfindung den als Median Mass Aerodynamic Diameter (MMAD) bekannten mittleren (Massenmittel) aerodynamischen Teilchendurchmesser bezeichnet.

Die als Träger verwendeten Salze von Cromoglicinsäure oder Nedocromil können in den erfindungsgemässen Aerosolformulierungen vorzugsweise ebenfalls in suspendierter Form mit einem mittleren Teilchendurchmesser von weniger als 6 µm (vorzugsweise mindestens etwa 1 µm) vorliegen. Sie können in an sich bekannter Weise, entweder allein oder zusammen mit dem pharmazeutischen Wirkstoff bzw. den pharmazeutischen Wirkstoffen, auf die gewünschte Teilchengrösse mikronisiert und im Treibmittel suspendiert werden. Gewünschtenfalls können die Cromoglicinsäure- und Nedocromilsalze aber auch in grösserer Teilchengrösse verwendet werden, wenn erwünscht ist, dass diese Salze nicht abgesprüht werden oder nicht in die Lunge gelangen.

Als Träger eignen sich grundsätzlich alle pharmazeutisch annehmbaren Salze von Cromoglicinsäure oder von Nedocromil, in denen eine oder beide Carboxylgruppen in deprotonierter, d.h. anionischer Form vorliegen. Bevorzugt verwendbar sind die Alkalimetallsalze und die Erdalkalimetallsalze, insbesondere die Natrium- und Kaliumsalze, wobei Dinatriumcromoglykat und Nedocromil-Natrium besonders bevorzugt sind.

Dinatriumcromoglykat und Nedocromil-Natrium werden - wie oben erwähnt - in bekannten Dosieraersolen in therapeutisch oder prophylaktisch wirksamer Menge von üblicherweise 1 mg bzw. 2 mg pro Sprühstoss verwendet. Demgegenüber werden die pharmazeutisch annehmbaren Salze von Cromoglicinsäure und von Nedocromil erfindungsgemäss nicht als therapeutische oder prophylaktische Wirkstoffe, sondern bloss als Träger eingesetzt und dementsprechend nur in Mengen verwendet, denen keine signifikante therapeutische oder prophylaktische Wirkung zukommt. Die erfindungsgemäss verwendeten Mengen an Cromoglicinsäuresalzen bzw. Nedocromilsalzen liegen daher in der Regel nicht über 500 µg pro Sprühstoss, wobei im allgemeinen Mengen von etwa 5 bis 250 µg, insbesondere etwa 10 bis 100 µg, pro Sprühstoss bevorzugt sind. Der Anteil an Cromoglicinsäuresalzen bzw. Nedocromilsäuresalzen in den erfindungsgemässen Aerosolformulierungen liegt daher in der Regel nicht über etwa 0,7 Gew.-% und beträgt vorzugsweise etwa 0,007 bis 0,36 Gew.-%, insbesondere etwa 0,015 bis 0,15 Gew.-%, bezogen auf die Gesamtformulierung.

Bezogen auf den suspendierten Wirkstoff, kann der Anteil an Cromoglicinsäure- und Nedocromilsalzen in einem relativ breiten Bereich variieren. Im allgemeinen beträgt jedoch das Gewichtsverhältnis der Cromoglicinsäure- und/oder Nedocromilsalze zum suspendierten pharmazeutischen Wirkstoff bzw. zu den suspendierten pharmazeutischen Wirkstoffen etwa 10:1 bis etwa 1:10, vorzugsweise etwa 5:1 bis etwa 1:5.

Vorzugsweise kann das Cromoglicinsäure- und/oder Nedocromilsalz im Vergleich zum pharmazeutischen Wirkstoff so ausgewählt werden, dass die Dichte dieser Materialien insgesamt mit der Dichte des Treibmittels vergleichbar ist. Beispielsweise kann mikronisiertes Formoterol-fumarat, das in HFA 227 zum Flotieren neigt, mit Dinatriumcromoglykat, das zum Sedimentieren neigt, kombiniert werden, um das suspendierte Material besser in Schwebe zu halten und Flotation oder Sedimentation zu minimieren.

Als nicht toxische flüssige Treibmittel für die erfindungsgemässen Aerosolformulierungen eignen sich grundsätzlich alle in Dosieraerosole üblicherweise verwendbaren druckverflüssigten Treibgase, beispielsweise Fluorchlorkohlenwasserstoffe wie Trichlormonofluormethan (F11), Dichlordifluormethan (F12), Monochlortrifluormethan (F13), Dichlormonofluormethan (F21), Monochlordifluormethan (F22), Monochlormonofluormethan (F31), 1,1,2-Trichlor-1,2,2-trifluorethan (F113), 1,2-Dichlor-1,1,2,2-tetrafluorethan (F114), 1-Chlor-1,1,2,2,2-pentafluorethan (F115), 2,2-Dichlor-1,1,1-trifluorethan (F123), 1,2-Dichlor-1,1,2-trifluorethan (F123a), 2-Chlor-1,1,1,2-tetrafluorethan (F124), 2-Chlor-1,1,2,2-tetrafluorethan (F124a), 1,2-Dichlor-1,1-difluorethan (F132b), 1-Chlor-1,2,2-trifluorethan (F133), 2-Chlor-1,1,1-trifluorethan (F133a), 1,1-Dichlor-1-fluorethan (F141b) und 1-Chlor-1,1-difluorethan (F142b), Alkane wie Propan, Butan und Isobutan, fluorierte Alkane wie Octafluorpropan (F218) und insbesondere Hydrofluoralkane wie Difluormethan (HFA 32), Pentafluorethan (HFA 125), 1,1,2,2-Tetrafluorethan (HFA 134), 1,1,1,2-Tetrafluorethan (HFA 134a), 1,1,2-Trifluorethan (HFA 143), 1,1,1-Trifluorethan (HFA 143a), Difluorethan (HFA 152a), 1,1,1,2,3,3,3-Heptafluorpropan (HFA 227) und dergleichen.

Bevorzugte Treibmittel sind die Hydrofluoralkane der allgemeinen Formel

CₓH_{y}F_{z} (I)

worin x für die Zahl 1, 2 oder 3 steht, y und z je eine ganze Zahl ≥ 1 bedeuten und y + z = 2x + 2 ist. Besonders geeignet sind in der Regel jene Hydrofluroalkane der Formel I, worin x für die Zahl 2 oder 3 steht.

Besonders bevorzugt sind Aerosolformulierungen, die HFA 134 oder HFA 227 oder Gemische dieser beiden Treibgase enthalten. HFA 134a und HFA 227 besitzen einen Dampfdruck von ca. 6 bar bzw. ca. 4,2 bar bei 20°C. Beide Treibgase unterscheiden sich hinsichtlich ihrer Dichte (ca. 1,2 g/ml für HFA 134a und ca. 1,4 g/ml für HFA 227), was insofern von Bedeutung ist, als durch geeignete Wahl des Treibmittels oder Treibmittelgemisches dessen Dichte besser an die Dichte der suspendierten Substanzen angeglichen und damit letztere besser in Schwebe gehalten werden können. Gewünschtenfalls kann die Dichte des Treibmittels durch Zusatz von Cosolventien oder anderen Treibgasen, wie beispielsweise Ethanol, Diethylether, Propan, n-Butan oder Isobutan, weiter erniedrigt werden.

Die erfindungsgemässen Aerosolformulierungen können vorzugsweise ein oder mehrere Hydrofluoralkane der Formel I, besonders bevorzugt 1,1,1,2-Tetrafluorethan (HFA 134a) und/oder 1,1,1,2,3,3,3-Heptafluorpropan (HFA 227), enthalten, und ihr Anteil an der Gesamtformulierung kann vorzugsweise mindestens etwa 50 Gew.-% und besonders bevorzugt mindestens etwa 80 Gew.-% betragen. In der Regel ist es vorteilhaft, diese Treibmittel in einer Menge von 90 Gew.-% oder mehr einzusetzen.

Gewünschtenfalls können die erfindungsgemässen Aerosolformulierungen als weiteres Treibgas Stickstoff oder insbesondere Distickstoffmonoxid (Lachgas) und/oder Kohlendioxid in einer Menge von etwa 0,0001 bis 10 Gew.-% enthalten. Konzentrationen von etwa 0,01 bis 3 Gew.-% sind im allgemeinen bevorzugt und Konzentrationen von etwa 0,1 bis 1,0 Gew.-% besonders bevorzugt; höhere Konzentrationen sind in der Regel nur dann sinnvoll, wenn die Formulierung einen vergleichsweise hohen Anteil an Cosolventien enthält. Es wurde nämlich überraschenderweise gefunden, dass man Treibgase mit vorteilhafteren Eigenschaften erhalten kann, wenn man den üblichen Treibgasen, insbesondere den genannten Hydrofluoralkanen, eine geringe Menge an Distickstoffmonoxid und/oder Kohlendioxid zusetzt. Derartige Treibgasgemische zeigen- im Unterschied zu Distickstoffmonoxid und Kohlendioxid als alleinige Treibgase - bei zunehmender Entleerung nur eine geringfügige Abnahme des Binnendrucks im Behältnis, was deren Verwendung als Treibmittel für Dosieraerosole ermöglicht. Zudem wurde überraschenderweise festgestellt, dass die Zugabe von Distickstoffmonoxid und/oder Kohlendioxid die Suspendierung von pharmazeutischen Wirkstoffen erleichtert, womit eher auf die Zugabe von oberflächenaktiven Stoffen und/oder Cosolventien verzichtet oder zumindest deren Anteil verringert werden kann. Des weiteren wurde gefunden, dass durch Zugabe von Distickstoffmonoxid und/oder Kohlendioxid die unerwünschte Deposition von Wirkstoff im Oropharynx reduziert und gleichzeitig die Fine Particle Dose erhöht werden kann. Ferner kann man durch Zugabe dieser Treibgase Sauerstoff aus den Hydrofluoralkanen oder anderen Treibmitteln verdrängen, was die Lagerstabilität von oxidationsempfindlichen Wirkstoffen verbessert, und je nach Menge an Distickstoffmonoxid und/oder Kohlendioxid den Binnendruck im Aerosolbehältnis so einstellen, wie es für die jeweilige Anwendung am sinnvollsten ist.

Im allgemeinen sind Aerosolformulierungen bevorzugt, die bei 20°C einen Druck von etwa 3 bis 10 bar, insbesondere etwa 3,5 bis 6 bar, aufweisen. Bei Verwendung von Cosolventien oder Treibmitteln mit niedrigem Dampfdruck kann ein allenfalls niedrigerer Druck vorzugsweise durch Zugabe von Distickstoffmonoxid und/oder Kohlendioxid entsprechend erhöht werden.

Die Herstellung der erfindungsgemässen Aerosolformulierungen kann in an sich bekannter Weise dadurch erfolgen, dass man den mikronisierten pharmazeutischen Wirkstoff und ein pharmazeutisch annehmbares Salz von Cromoglicinsäure und/oder Nedocromil dem Treibmittel zusetzt und, gewünschtenfalls, Distickstoffmonoxid und/oder Kohlendioxid unter Druck einleitet. Diese Schritte können grundsätzlich in beliebiger Reihenfolge durchgeführt werden. Bei Verwendung von Distickstoffmonoxid und/oder Kohlendioxid ist es jedoch in der Regel bevorzugt, zuerst dieses in das Treibmittel einzuleiten und dann den mikronisierten Wirkstoff und das Cromoglicinsäure- und/oder Nedocromilsalz zuzusetzen. Die Formulierungen können unter Verwendung von üblichen Rührern und Homogenisatoren hergestellt werden. Zur Abfüllung können bekannte Verfahren, wie die Kalt- oder Druckfülltechnik oder Modifikationen dieser Techniken, eingesetzt werden. Als Behältnisse eignen sich beispielsweise drucksichere Behälter aus Glas, Kunststoff oder Aluminium, die mit Dosierventilen von z.B. 10 bis 140 µl bestückt und mit handelsüblichen - auch atemzuggetriggerten - Mundrohradaptern versehen werden können.

Obwohl sich der Zusatz von Cosolventien und oberflächenaktiven Mittel infolge der Verwendung von Cromoglicinsäure- und/oder Nedocromilsalzen und allenfalls infolge der Verwendung von Distickstoffmonoxid und/oder Kohlendioxid meist erübrigt, kann die Zugabe einer geringen Menge an Cosolvens gelegentlich von Vorteil sein. Als Cosolventien eignen sich beispielsweise Wasser, Alkohole mit 1 bis 3 Kohlenstoffatomen, Alkane mit 3 bis 6 Kohlenstoffatomen und Dialkylether mit 2 bis 4 Kohlenstoffatomen. Beispiele bevorzugter Cosolventien sind: Ethanol, Propanol, Isopropanol, Ethylenglykol, Propylenglykol, Glycerin, Propan, Butan, Isobutan, Pentan, Dimethylether und Diethylether, wobei Ethanol, Glycerin, Propylenglykol und Diethylether oder deren Gemische und insbesondere Ethanol in der Regel besonders bevorzugt sind. Bevorzugte Cosolvensgemische, mit denen zugleich eine Gleitmittelwirkung erreicht wird, sind Ethanol zusammen mit Glycerin und/oder Propylenglykol sowie Diethylether zusammen mit Glycerin und/oder Propylenglykol. Im allgemeinen liegt der Anteil an Cosolventien, falls vorhanden, nicht über etwa 15 Gew.-%, vorzugsweise nicht über etwa 10 Gew.-% und meist nicht über etwa 5 Gew.-%, bezogen auf die Gesamtformulierung.

Die erfindungsgemässen Aerosolformulierungen können vorzugsweise im wesentlichen frei von oberflächenaktiven Mitteln sein, d.h. vorzugsweise weniger als etwa 0,0001 Gew.-% an oberflächenaktiven Mitteln enthalten. Gewünschtenfalls können sie jedoch oberflächenaktive Mittel wie Ölsäure, Lecithin, Sorbitantrioleat, Cetylpyridiniumchlorid, Benzalkoniumchlorid, Polyoxyethylen(20)sorbitanmonolaurat, Polyoxyethylen(20)sorbitanmonostearat, Polyoxyethylen(20)-sorbitanmonooleat, Polyoxypropylen-polyoxyethylen-Blockcopolymere, Polyoxypropylen-polyoxyethylen-ethylendiamin-Blockcopolymere, ethoxyliertes Ricinusöl und dergleichen enthalten, wobei der Anteil an oberflächenaktiven Mitteln, falls vorhanden, vorzugsweise etwa 0,0001 bis 1 Gew.-%, insbesondere etwa 0,001 bis 0,1 Gew.-%, bezogen auf die Gesamtformulierung, betragen kann.

Weiterhin können die erfindungsgemässen Aerosolformulierungen gewünschtenfalls Puffersubstanzen oder Stabilisatoren wie Citronensäure, Ascorbinsäure, Natrium-EDTA, Vitamin E, N-Acetylcystein und dergleichen enthalten. Im allgemeinen werden solche Substanzen, falls vorhanden, in Mengen von nicht mehr als etwa 1 Gew.-%, beispielsweise in einer Menge von etwa 0,0001 bis 1 Gew.-%, bezogen auf die Gesamtformulierung, verwendet.

Die erfindungsgemässe Verwendung von Cromoglicinsäure- und/oder Nedocromilsalzen gestattet somit die Herstellung verbesserter Suspenionsaerosolformulierungen von pharmazeutischen Wirkstoffen, insbesondere von niedrig dosierten Wirkstoffen. Die Erfindung betrifft daher ebenfalls die Verwendung eines festen, pharmazeutisch annehmbaren Salzes von Cromoglicinsäure und/oder Nedocromil in therapeutisch und propylaktisch unwirksamer Menge in einer medizinischen Suspensionsaerosolformulierung zur Reduzierung unerwünschter Adsorption bzw. zur Verbesserung der Dosiergenauigkeit und/oder Verringerung der Feuchtigkeitsempfindlichkeit eines suspendierten pharmazeutischen Wirkstoffes.

Mit der erfindungsgemässen Formulierungstechnologie ist es also möglich, Wirkstoffe oder Wirkstoffkombinationen als Dosieraerosole mit vorteilhafteren Eigenschaften herzustellen, wie nachfolgend anhand einiger Beispiele weiter veranschaulicht wird.

### Beispiel 1

6 g mikronisiertes Formoterol-fumarat und 12 g mikronisiertes Dinatriumcromoglykat werden in einen Druckansatzkessel eingewogen. Nach Verschliessen und Evakuieren des Ansatzkessels werden unter Rühren je 35 kg HFA 134a und HFA 227 zugegeben, die zuvor in einem anderen Druckansatzkessel mit 3 Gew.-% Ethanol versetzt wurden. Nach Homogenisieren wird die erhaltene Suspension mittels Druckfülltechnik in mit Dosierventilen verschlossene Aluminiumdosen abgefüllt.

### Beispiel 2

2 g mikronisiertes Ipratropiumbromid und 10 g mikronisiertes Dinatriumcromoglykat werden gemischt und in einen Druckansatzkessel eingewogen. Nach Verschliessen und Evakuieren des Ansatzkessels werden 10 kg HFA 227 zugegeben, das zuvor in einem anderen Druckansatzkessel mit Kohlendioxid begast und auf einen Druck von 5 bar bei 20°C eingestellt wurde. Nach Homogenisieren dieser Mischung wird die erhaltene Suspension mittels Druckfülltechnik in mit Dosierventilen verschlossene Aluminiumdosen abgefüllt.

### Beispiel 3

2,5 g mikronisiertes Glycopyrroniumbromid und 2,5 g mikronisiertes Nedocromil-Natrium werden gemischt und in einen Druckansatzkessel eingewogen. Nach Verschliessen und Evakuieren des Ansatzkessels werden 10,5 kg eines Treibgasgemisches von HFA 227 und HFA 134a (Gewichtsverhältnis 90:10) zugegeben, das zuvor in einem anderen Druckansatzkessel mit 1 Gew.-% Ethanol versetzt und mit Distickstoffoxid begast und auf einen Druck von 5,5 bar bei 20°C eingestellt wurde. Nach Homogenisieren dieser Mischung wird die erhaltene Suspension mittels Druckfülltechnik in mit Dosierventilen verschlossene Aluminiumdosen abgefüllt.

### Beispiel 4

Eine Pulvermischung, bestehend aus 5 g mikronisiertem Formoterol-fumarat, 20 g mikronisiertem Glycopyrroniumbromid und 25 g mikronisiertes Dinatriumcromoglykat, wird in einen Druckansatzkessel eingewogen. Nach Verschliessen und Evakuieren des Ansatzkessels werden unter Rühren 70 kg HFA 227 zugegeben, das zuvor in einem anderen Druckansatzkessel mit 2 Gew.-% Ethanol versetzt und mit Distickstoffoxid begast und auf einen Druck von 5 bar bei 20°C eingestellt wurde. Nach Homogenisieren wird die erhaltene Suspension mittels Druckfülltechnik in mit Dosierventilen verschlossene Aluminiumdosen abgefüllt.

### Beispiel 5

2,5 g mikronisiertes Glycopyrroniumbromid, 5 g mikronisiertes Levalbuterol und 5 g mikronisiertes Nedocromil-Natrium werden in einen Druckansatzkessel eingewogen. Nach Verschliessen und Evakuieren des Ansatzkessels werden 10,5 kg eines Treibgasgemisches von HFA 227 und HFA 134a (Gewichtsverhältnis 80:20) zugegeben, das zuvor in einem anderen Druckansatzkessel mit Distickstoffoxid begast und auf einen Druck von 5,25 bar bei 20°C eingestellt wurde. Nach Homogenisieren dieser Mischung wird die erhaltene Suspension mittels Druckfülltechnik in mit Dosierventilen verschlossene Aluminiumdosen abgefüllt.

### Beispiel 6

5 g mikronisiertes Formoterol-fumarat, 30 g mikronisiertes Fluticason-propionat und 10 g mikronisiertes Dinatriumcromoglykat werden in einen Druckansatzkessel eingewogen. Nach Verschliessen und Evakuieren des Ansatzkessels werden unter Rühren 70 kg HFA 227 zugegeben, das zuvor in einem anderen Druckansatzkessel mit 2 Gew.-% Ethanol versetzt und mit Distickstoffoxid begast und auf einen Druck von 5 bar bei 20°C eingestellt wurde. Nach Homogenisieren wird die erhaltene Suspension mittels Druckfülltechnik in mit Dosierventilen verschlossene Aluminiumdosen abgefüllt.

### Beispiel 7

20 g mikronisiertes Tiotropiumbromid und 10 g mikronisiertes Nedocromil-Natrium werden in einen Druckansatzkessel eingewogen. Nach Verschliessen und Evakuieren des Ansatzkessels werden unter Rühren 70 kg eines Treibgasgemisches von HFA 227 und HFA 134a (Gewichtsverhältnis 70:30) zugegeben, das zuvor in einem anderen Druckansatzkessel mit 0,5 Gew.-% Ethanol versetzt wurde. Nach Homogenisieren wird die erhaltene Suspension mittels Druckfülltechnik in mit Dosierventilen verschlossene Aluminiumdosen abgefüllt.

### Beispiel 8

3 g mikronisiertes Glycopyrroniumbromid, 3 g mikronisiertes Salmeterol-xinafoat und 3 g mikronisiertes Dinatriumcromoglykat werden gemischt und in einen Druckansatzkessel eingewogen. Nach Verschliessen und Evakuieren des Ansatzkessels werden 10,5 kg eines Treibgasgemisches von HFA 227 und HFA 134a (Gewichtsverhältnis 75:25) zugegeben, das zuvor in einem anderen Druckansatzkessel mit Distickstoffoxid begast und auf einen Druck von 5,25 bar eingestellt wurde. Nach Homogenisieren dieser Mischung wird die erhaltene Suspension mittels Druckfülltechnik in mit Dosierventilen verschlossene Aluminiumdosen abgefüllt.

### Beispiel 9

Eine Pulvermischung, bestehend aus 10 g mikronisiertem Budesonid und 1 g mikronisiertem Dinatriumcromoglykat, wird in einen Druckansatzkessel eingewogen. Nach Verschliessen und Evakuieren des Ansatzkessels werden unter Rühren 7 kg eines Treibgasgemisches von HFA 227 und HFA 134a (Gewichtsverhältnis 65:35) zugegeben, das zuvor in einem anderen Druckansatzkessel mit 1 Gew.-% Ethanol versetzt wurde. Nach Homogenisieren wird die erhaltene Suspension mittels Druckfülltechnik in mit Dosierventilen verschlossene Aluminiumdosen abgefüllt.

### Beispiel 10

0,5 g mikronisiertes Formoterol-fumarat und 2,0 g mikronisiertes Dinatriumcromoglykat werden in einen Druckansatzkessel eingewogen. Nach Verschliessen und Evakuieren des Ansatzkessels werden unter Rühren 7,0 kg HFA 227 zugegeben, das zuvor in einem anderen Druckansatzkessel mit 3 Gew.-% Ethanol und 0,02 Gew.-% Glycerin versetzt und mit Distickstoffoxid begast und auf einen Druck von 5 bar bei 20°C eingestellt wurde. Nach Homogenisieren wird die erhaltene Suspension mittels Druckfülltechnik in mit Dosierventilen verschlossene Aluminiumdosen abgefüllt.

### Beispiel 11

Eine Pulvermischung, bestehend aus 10 g mikronisiertem Budesonid, 0,5 g mikronisiertem Formoterol-tartrat und 1 g Dinatriumcromoglykat, wird in einen Druckansatzkessel eingewogen. Nach Verschliessen und Evakuieren des Ansatzkessels werden unter Rühren 70 kg HFA 227 zugegeben, das zuvor in einem anderen Druckansatzkessel mit 2 Gew.-% Ethanol und 0,2 Gew.-% Propylenglykol versetzt wurde. Nach Homogenisieren wird die erhaltene Suspension mittels Druckfülltechnik in mit Dosierventilen verschlossene Aluminiumdosen abgefüllt.

### Beispiel 12

Eine Pulvermischung, bestehend aus 0,5 g mikronisiertem Formoterol-tartrat, 10 g mikronisiertem Fluticason-dipropionat und 1 g mikronisiertem Dinatriumcromoglykat, wird in einen Druckansatzkessel eingewogen. Nach Verschliessen und Evakuieren des Ansatzkessels werden unter Rühren 7 kg HFA 227 zugegeben, das zuvor in einem anderen Druckansatzkessel mit 2 Gew.-% Diethylether und je 0,02 Gew.-% Benzalkoniumchlorid, Citronensäure und Propylenglykol versetzt wurde. Nach Homogenisieren wird die erhaltene Suspension mittels Druckfülltechnik in mit Dosierventilen verschlossene Aluminiumdosen abgefüllt.

### Beispiel 13

3 g mikronisiertes Glycopyrroniumbromid, 3 g mikronisiertes Salmeterol-xinafoat, 3 g mikronisiertes Dinatriumcromoglykat, 0,03 g Natrium-EDTA und 0,03 g Glycerin werden gemischt und in einen Druckansatzkessel eingewogen. Nach Verschliessen und Evakuieren des Ansatzkessels werden 10,5 kg eines Treibgasgemisches von HFA 227 und HFA 134a (Gewichtsverhältnis 75:25) zugegeben, das zuvor in einem anderen Druckansatzkessel mit Distickstoffoxid begast und auf einen Druck von 5,25 bar eingestellt wurde. Nach Homogenisieren dieser Mischung wird die erhaltene Suspension mittels Druckfülltechnik in mit Dosierventilen verschlossene Aluminiumdosen abgefüllt.

## Patentansprüche

1. Medizinische Aerosolformulierung, umfassend ein festes, pharmazeutisch annehmbares Salz von Cromoglicinsäure und/oder Nedocromil in therapeutisch und prophylaktisch unwirksamer Menge im Bereich von 5 bis 250 µg pro Sprühstoss, eine wirksame Menge eines davon verschiedenen, fein verteilten pharmazeutischen Wirkstoffes mit einem mittleren Teilchendurchmesser von weniger als 6 µm und ein nicht toxisches flüssiges Treibmittel, in dem der Wirkstoff in suspendierter Form vorliegt.

2. Aerosolformulierung nach Anspruch 1, **dadurch gekennzeichnet, dass** das Salz von Cromoglicinsäure und/oder Nedocromil ein Alkalimetallsalz oder ein Erdalkalimetallsalz, vorzugsweise ein Natrium- oder Kaliumsalz, ist.

3. Aerosolformulierung nach Anspruch 1 oder 2, **dadurch gekennzeichnet, dass** das Salz von Cromoglicinsäure und/oder Nedocromil Dinatriumcromoglykat oder Nedocromil-Natrium ist.

4. Aerosolformulierung nach einem der Ansprüche 1 bis 3, **dadurch gekennzeichnet, dass** das Salz von Cromoglicinsäure und/oder Nedocromil in suspendierter Form mit einer Teilchengrösse von weniger als 6 µm vorliegt.

5. Aerosolformulierung nach einem der Ansprüche 1 bis 4, **dadurch gekennzeichnet, dass** das Salz von Cromoglicinsäure oder Nedocromil in einer Menge von 10 bis 100 µg, pro Sprühstoss vorliegt.

6. Aerosolformulierung nach einem der Ansprüche 1 bis 5, **dadurch gekennzeichnet, dass** das Cromoglicinsäure- und/oder Nedocromilsalz und der suspendierte pharmazeutische Wirkstoff im Gewichtsverhältnis von 10:1 bis 1:10, vorzugsweise 5:1 bis 1:5, vorliegen.

7. Aerosolformulierung nach einem der Ansprüche 1 bis 6, **dadurch gekennzeichnet, dass** sie als pharmazeutischen Wirkstoff ein Betamimetikum, Anticholinergikum, Antiallergikum oder einen entzündungshemmenden Wirkstoff enthält.

8. Aerosolformulierung nach einem der Ansprüche 1 bis 7, **dadurch gekennzeichnet, dass** sie als pharmazeutischen Wirkstoff Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid oder ein pharmazeutisch annehmbares Salz oder Derivat davon enthält.

9. Aerosolformulierung nach einem der Ansprüche 1 bis 8, **dadurch gekennzeichnet, dass** der suspendierten Wirkstoff in einer Menge von 0,0001 bis 0,2 Gew.-%, vorzugsweise höchstens 0,1 Gew.-%, vorliegt.

10. Aerosolformulierung nach einem der Ansprüche 1 bis 9, **dadurch gekennzeichnet, dass** sie als pharmazeutische Wirkstoffe Fluticason, Ipratropium, Oxitropium, Glycopyrronium, Tiotropium, Budesonid, Mometason, Ciclesonid, Rofleponid oder eine pharmazeutisch annehmbares Salz oder Derivat davon in Kombination mit Levalbuterol, Formoterol und/oder Salmeterol oder einem pharmazeutisch annehmbaren Derivat davon enthält.

11. Aerosolformulierung nach einem der Ansprüche 1 bis 10, **dadurch gekennzeichnet, dass** sie als Treibmittel ein oder mehrere Hydrofluoralkane der allgemeinen Formel
CₓH_{y}F_{z} (I)
worin x für die Zahl 1, 2 oder 3 steht, y und z je eine ganze Zahl ≥ 1 bedeuten und y + z = 2x + 2 ist, enthält.

12. Aerosolformulierung nach einem der Ansprüche 1 bis 11, **dadurch gekennzeichnet, dass** sie als Treibmittel 1,1,1,2-Tetrafluorethan und/oder 1,1,1,2,3,3,3-Heptafluorpropan enthält.

13. Aerosolformulierung nach einem der Ansprüche 1 bis 12, **dadurch gekennzeichnet, dass** sie Distickstoffmonoxid und/oder Kohlendioxid in einer Menge von 0,0001 bis 10 Gew.-% enthält.

14. Aerosolformulierung nach einem der Ansprüche 1 bis 13, **dadurch gekennzeichnet, dass** sie bei 20°C einen Druck von 3 bis 10 bar, vorzugsweise 3,5 bis 6 bar, aufweist.

15. Aerosolformulierung nach einem der Ansprüche 1 bis 14, **dadurch gekennzeichnet, dass** sie ein Cosolvens, vorzugsweise Ethanol, Glycerin, Propylenglykol und/oder Diethylether, enthält.

16. Aerosolformulierung nach Anspruch 15, **dadurch gekennzeichnet, dass** sie höchstens 15 Gew.-%, vorzugsweise höchstens 10 Gew.-%, an Cosolvens enthält.

17. Aerosolformulierung nach einem der Ansprüche 1 bis 16, **dadurch gekennzeichnet, dass** sie im wesentlichen frei von oberflächenaktiven Mitteln ist.

18. Verwendung eines festen, pharmazeutisch annehmbaren Salzes von Cromoglicinsäure und/oder Nedocromil in therapeutisch und prophylaktisch unwirksamer Menge in einer medizinischen Suspensionsaeorosolformulierung zur Verbesserung der Dosiergenauigkeit und/oder Verringerung der Feuchtigkeitsempfindlichkeit eines suspendierten pharmazeutischen Wirkstoffes.

## Claims

1. A medicinal aerosol formulation, comprising a solid, pharmaceutically acceptable salt of cromoglycic acid and/or nedocromil in a therapeutically and prophylactically inefficacious amount in the range from 5 to 250 µg per puff of spray, an efficacious amount of a finely divided pharmaceutically active compound which is different therefrom, having an mean particle diameter of less than 6 µm, and a non-toxic liquid propellant, in which the active compound is present in suspended form.

2. The aerosol formulation as claimed in claim 1, wherein the salt of cromoglycic acid and/or nedocromil is an alkali metal salt or an alkaline earth metal salt, preferably a sodium or potassium salt.

3. The aerosol formulation as claimed in claim 1 or 2, wherein the salt of cromoglycic acid and/or nedocromil is disodium cromoglycate or nedocromil sodium.

4. The aerosol formulation as claimed in one of claims 1 to 3, wherein the salt of cromoglycic acid and/or nedocromil is present in suspended form having a particle size of less than 6 µm.

5. The aerosol formulation as claimed in one of claims 1 to 4, wherein the salt of cromoglycic acid or nedocromil is present in an amount of from 10 to 100 µg, per puff of spray.

6. The aerosol formulation as claimed in one of claims 1 to 5, wherein the cromoglycic acid and/or nedocromil salt and the suspended pharmaceutically active compound are present in the weight ratio from 10:1 to 1:10, preferably 5:1 to 1:5.

7. The aerosol formulation as claimed in one of claims 1 to 6, which as pharmaceutically active compound contains a betamimetic, anticholinergic, antiallergic or an antiinflammatory active compound.

8. The aerosol formulation as claimed in one of claims 1 to 7, which as pharmaceutically active compound contains formoterol, salmeterol, fenoterol, clenbuterol, levalbuterol, ipratropium, oxitropium, glycopyrronium, tiotropium, budesonide, ciclesonide, mometasone, fluticasone, beclomethasone, flunisolide, loteprednol, triamcinolone, amiloride, rofleponide or a pharmaceutically acceptable salt or derivative thereof.

9. The aerosol formulation as claimed in one of claims 1 to 8, wherein the suspended active compound is present in an amount of from 0.0001 to 0.2% by weight, preferably at most 0.1% by weight.

10. The aerosol formulation as claimed in one of claims 1 to 9, which contains as pharmaceutically active compounds fluticasone, ipratropium, oxitropium, glycopyrronium, tiotropium, budesonide, mometasone, ciclesonide, rofleponide or a pharmaceutically acceptable salt or derivative thereof in combination with a levalbuterol, formoterol and/or salmeterol or a pharmaceutically acceptable derivative thereof.

11. The aerosol formulation as claimed in one of claims 1 to 10, which as propellant contains one or more hydrofluoroalkanes of the general formula
CₓH_{y}F_{z} (I)
in which x is 1, 2 or 3, y and z are each an integer ≥ 1 and y + z = 2x + 2.

12. The aerosol formulation as claimed in one of claims 1 to 11, which as propellant contains 1,1,1,2-tetrafluoroethane and/or 1,1,1,2,3,3,3-heptafluoropropane.

13. The aerosol formulation as claimed in one of claims 1 to 12, which contains dinitrogen monoxide and/or carbon dioxide in an amount of from 0.0001 to 10% by weight.

14. The aerosol formulation as claimed in one of claims 1 to 13, which has a pressure of 3 to 10 bar, preferably 3.5 to 6 bar, at 20°C.

15. The aerosol formulation as claimed in one of claims 1 to 14, which contains a cosolvent, preferably ethanol, glycerol, propylene glycol and/or diethyl ether.

16. The aerosol formulation as claimed in claim 15, which contains at most 15% by weight, preferably at most 10% by weight, of cosolvent.

17. The aerosol formulation as claimed in one of claims 1 to 16, which is essentially free of surface-active agents.

18. The use of a solid, pharmaceutically acceptable salt of cromoglycic acid and/or nedocromil in a therapeutically and prophylactically inefficacious amount in a medicinal suspension aerosol formulation for improving the dosage accuracy and/or reducing the moisture sensitivity of a suspended pharmaceutically active compound.

## Revendications

1. Formulation médicale d'aérosol, comprenant un sel pharmaceutiquement acceptable solide de l'acide cromoglycinique et/ou du nédocromil en une quantité thérapeutiquement et prophylactiquement inefficace dans la plage de 5 à 250 µg par bouffée pulvérisée, une quantité efficace d'une substance active pharmaceutique différente de celui-ci, finement divisée, présentant un diamètre moyen des particules inférieur à 6 µm et un agent propulseur liquide non toxique dans lequel la substance active sous trouve sous forme suspendue.

2. Formulation d'aérosol selon la revendication 1, **caractérisée en ce que** le sel de l'acide cromoglycinique et/ou du nédocromil est un sel de métal alcalin ou de métal alcalino-terreux, de préférence un sel de sodium ou de potassium.

3. Formulation d'aérosol selon les revendications 1 ou 2, **caractérisée en ce que** le sel de l'acide cromoglycinique et/ou du nédocromil est le glycate disodique ou nédocromil sodique.

4. Formulation d'aérosol selon l'une quelconque des revendications 1 à 3, **caractérisée en ce que** le sel de l'acide cromoglycinique et/ou du nédocromil se trouve sous forme suspendue avec une grosseur de particules inférieure à 6 µm.

5. Formulation d'aérosol selon l'une quelconque des revendications 1 à 4, **caractérisée en ce que** le sel de l'acide cromoglycinique ou du nédocromil se trouve en une quantité de 10 à 100 µg par bouffée pulvérisée.

6. Formulation d'aérosol selon l'une quelconque des revendications 1 à 5, **caractérisée en ce que** le sel de l'acide cromoglycinique et/ou du nédocromil et la substance active pharmaceutique mise en suspension se trouvent dans un rapport pondéral de 10:1 à 1:10, de préférence de 5:1 à 1:5.

7. Formulation d'aérosol selon l'une quelconque des revendications 1 à 6, **caractérisée en ce qu'**elle contient comme substance active pharmaceutique un bêta-mimétique, un anticholinergique, un anti-allergique ou une substance active anti-inflammatoire.

8. Formulation d'aérosol selon l'une quelconque des revendications 1 à 7, **caractérisée en ce qu'**elle contient comme substance active pharmaceutique du formotérol, du salmétérol, du fénotérol, du clenbutérol, du lévalbutérol, de l'ipratropium, de l'oxitropium, du glycopyrronium, du tiotropiom, du budésonide, du ciclésonide, du mométasone, du fluticasone, du béclométhasone, du flunisolide, du lotéprednol, de la triamcinolone, de l'amiloride, du rofléponide ou un sel pharmaceutiquement acceptable ou un dérivé de ceux-ci.

9. Formulation d'aérosol selon l'une quelconque des revendications 1 à 8, **caractérisée en ce que** la substance active mise en suspension se trouve en une quantité de 0,0001 à 0,2% en poids, de préférence d'au maximum 0,1% en poids.

10. Formulation d'aérosol selon l'une quelconque des revendications 1 à 9, **caractérisée** ce qu'elle contient comme substance active pharmaceutique du fluticasone, de l'ipratropium, de l'oxitropium, du glycopyrronium, du tiotropium, du budésonide, du mométasone, du ciclosonide, du rofléponide ou un sel ou un dérivé pharmaceutiquement acceptable de ceux-ci en combinaison avec du lévalbutérol, du formotérol et/ou du salmétérol ou un dérivé pharmaceutiquement acceptable de ceux-ci.

11. Formulation d'aérosol selon l'une quelconque des revendications 1 à 10, **caractérisée en ce qu'**elle contient comme agent propulseur un ou plusieurs hydrofluoroalcanes de formule générale
CₓH_{y}F_{z} (I)
dans laquelle x représente le nombre 1, 2 ou 3, y et z sont à chaque fois un nombre entier ≥ 1 et y+z = 2x+2.

12. Formulation d'aérosol selon l'une quelconque des revendications 1 à 11, **caractérisée en ce qu'**elle contient comme agent propulseur du 1,1,1,2-tétrafluoroéthane et/ou du 1,1,1,2,3,3,3-heptafluoropropane.

13. Formulation d'aérosol selon l'une quelconque des revendications 1 à 12, **caractérisée en ce qu'**elle contient du protoxyde d'azote et/ou du dioxyde de carbone en une quantité de 0,0001 à 10% en poids.

14. Formulation d'aérosol selon l'une quelconque des revendications 1 à 13, **caractérisée en ce qu'**elle présente à 20°C une pression de 3 à 10 bars, de préférence de 3,5 à 6 bars.

15. Formulation d'aérosol selon l'une quelconque des revendications 1 à 14, **caractérisée en ce qu'**elle contient un cosolvant, de préférence de l'éthanol, du glycérol, du propylèneglycol et/ou du diéthyléther.

16. Formulation d'aérosol selon la revendication 15, **caractérisée en ce qu'**elle contient au maximum 15% en poids, de préférence au maximum 10% en poids, de cosolvant.

17. Formulation d'aérosol selon l'une quelconque des revendications 1 à 16, **caractérisée en ce qu'**elle est essentiellement exempte d'agents tensioactifs.

18. Utilisation d'un sel solide, pharmaceutiquement acceptable de l'acide cromoglycinique et/ou du nédocromil en une quantité thérapeutiquement et prophylactiquement inefficace dans une formulation médicale d'aérosol à suspension pour l'amélioration de la précision de dosage et/ou la diminution de la sensibilité à l'humidité d'une substance active pharmaceutique en suspension.
